# EUROPÄISCHE PATENTSCHRIFT

(11) **EP 0 373 584 B1**
(45) Veröffentlichungstag und Bekanntmachung des Hinweises auf die Patenterteilung: **02.03.1994**
(21) Anmeldenummer: 89122902.3
(22) Anmeldetag: 12.12.1989
(51) Int. Cl.: A61H 9/00

(54) **Massagegerät**
Massage device
Dispositif de massage

(30) Priorität: 12.12.1988 DE 3841793
(43) Veröffentlichungstag der Anmeldung: 20.06.1990
(73) Patentinhaber: AYK MED. TECHN. GERÄTE GMBH, D-51491 Overath (DE)
(72) Erfinder: Aydnik, Heinz, D-5963 Overath 6 (DE)
(74) Vertreter: König, Reimar, Dr.-Ing.

(56) Entgegenhaltungen:
- DE-A- 2 524 457
- DE-A- 3 443 780
- DE-U- 8 714 832
- US-A- 4 635 620

## Beschreibung

Die Erfindung betrifft ein Massagegerät mit einer Haube aus einer Hohlraumwandung, wovon die Innenwand mit Luftdüsen versehen ist.

Neben der Behandlung des Körpers durch bespielsweise planmäßiges Streichen, Reiben, Kneten und Klopfen ist es bekannt, die Haut und die darunterliegenden Weichteile, insbesondere die Muskulatur, mittelbar, d.h. ohne Handgriffe durch einen Masseur zu behandeln.

Bei einem aus der deutschen Offenlegungsschrift 34 43 780 bekannten Massagegerät geschieht dies mittels eines Fluids, wie Luft und insbesondere Wasser. Das Massagemedium wird mittels in einer Haube angeordneter Strahlrohre zugeführt, die zahlreiche Düsen aufweisen. Die Strahlrohre sind an ein gemeinsames Zuleitungs-bzw. Verteilerrohr angeschlossen und in einem durch Anschläge begrenzten Winkelbereich verschwenkbar, so daß der Auftreffpunkt der Wasserstrahlen auf den Körper ständig wechselt. Die in ihrem Inneren mit einer Liegefläche ausgestattete Haube ist über eine horitontal verschiebbare Schiebetüre zugänglich. Während der Massage ragt der Kopf des auf einer Liege ruhenden Patienten aus der Haube heraus und ruht auf einer Verlängerung der Liege. Eine elektronische Steuerung erlaubt es, den Behandlungsablauf vollautomatisch zu steuern, beispielsweise den Druck der Wasserstrahlen, die Wassertemperatur und die Behandlungsdauer festzulegen. Um eine Massage von Fuß bis Kopf zu ermöglichen, sind über die Länge der Haube mit Abstand parallel nebeneinander mehrere Strahlrohre in der Haube angeordnet; sie liegen völlig frei und ungeschützt unterhalb der Haubendecke.

Demgegenüber ist in der deutschen Offenlegungsschrift 25 24 457 eine Massagevorrichtung gemäß der eingangs genannten Art beschrieben, die wie das Massagegerät gemäß der deutschen Offenlegungsschrift 34 43 780 in einer Haube angeordnete Verteilerrohre und davon ausgehende Düsen besitzt. Diese Verteilerrohre und die Düsen sind durch eine Innenwand abgedeckt und liegen daher nicht wie bei dem Massagegerät gemäß der deutschen Offenlegungsschrift 34 43 780 frei im Innenraum des Massagegeräts. Die Verteilerrohre und die davon ausgehenden Düsen sind im Hohlraum einer doppelwandigen Haube angeordnet, während sie bei dem Massagegerät gemäß der deutschen Offenlegungsschrift 34 43 780 unterhalb der Haube freiliegen.

Der Erfindung liegt die Aufgabe zugrunde, bei einem gattungsgemäßen Massagegerät die Luftzuführung zu verbessern, insbesondere den Aufwand für die Luftzuführungen zu verringern.

Erfindungsgemäß wird diese Aufgabe dadurch gelöst, daß die Luftdüsen mit erwärmter Luft versorgt werden und die erwärmte Luft unmittelbar in den von der Hohlraumwandung eingeschlossenen Holraum der Haube geleitet wird. Die beispielsweise aus Leichtmetall oder vorzugsweise aus Kunststoff bestehende Haube läßt sich somit durch einfaches Aufbohren der Innenwand mit Luftdüsen versehen.

Bei einer Haube, die mit stirnseitigen Wänden versehen ist, von denen zumindest eine Stirnwand einen Ausschnitt besitzt, läßt sich ein Patient, der beispielsweise während der Behandlung stehen oder liegen kann, nahezu völlig einhüllen und damit die Wirkung des warmen Luftstroms verbessern; der durch den Ausschnitt in der Stirnwand der Haube herausragende Kopf des Patienten liegt während der Behandlung frei und ruht auf einer Unterlage.

Wenn die Haube aus mehreren Halbschalensegmenten besteht und gegebenenfalls jedes Segment mit einem separaten Luftanschluß versehen ist, lassen sich die Luftdüsen selektiv bzw. zonenweise ansteuern. Es kann dann nämlich der Bereich der Luftzufuhr beliebig variiert werden, indem nur die gewünschten Segmente mit Luft versorgt werden.

Wenn die Luftdüsen selektiv ansteuerbar, vorzugsweise elektronisch steuer- und/oder regelbar sind, ist ein variables Luftbestrahlen und damit ein variables Durchmassieren des Patienten, sei es aus medizinisch-therapeutischen Gründen oder zur Leistungssteigerung und Entmüdung bzw. nach dem beim sportlichen Wettkampf von Fuß bis Kopf möglich. Die entweder selektiv oder zonenweise gesteuerten, gegebenenfalls mit unterschiedlicher Intensität und während der Dauer der Massage veränderlicher Temperatur der Luft auf den Körper abgegebenen Luftströme ermöglichen es, die Muskulatur auf angenehme Art und Weise wirksam zu entspannen. Hinsichtlich der Lufttemperatur empfiehlt es sich, die Massage mit einer hohen Anfangstemperatur zu beginnen, um damit ein Öffnen der Poren zu erreichen, während zum Massageende hin die Temperatur abgesenkt werden sollte, so daß sich die Poren wieder schließen; ein Temperaturschock läßt sich damit vermeiden.

Die Wirkung der Luftmassage läßt sich durch in der Haube angeordnete Lichtstrahler unterstützen bzw. verbessern. Beispielsweise fördern Solar- oder Infrarotstrahler, vorzugsweise Niederdruckröhren, aber auch Hochdruckquarzbrenner, das Wohlbefinden der zu behandelnden Person.

Bei einer bevorzugten Ausführung der Erfindung ist die Haube schwenkbeweglich an einem Untergestell befestigt, das zur Aufnahme einer liegenden Person als Liegefläche entweder eine Matratze oder vorzugsweise ein Wasserbett aufweisen kann. Damit ist es einer zu behandelnden Person möglich, während der Dauer der Luftmassage eine angenehme Position einzunehmen, nämlich in einer ruhenden Liegelage, beispielsweise auf einem Wasserbett.

Bei einem ein Untergestell aufweisenden, erfindungsgemäßen Massagegerät läßt sich vorzugsweise in dem Untergestell zumindest ein Gebläse anordnen. Mittels des Gebläses kann, beispielsweise durch einen Filter vorgereinigte, Umgebungsluft angesaugt und den einzelnen Luftdüsen bzw. Zonen zugeführt werden. Eine Kompaktbauweise des Massagegerätes ergibt sich dann, wenn dem Gebläse in Richtung des Luftstroms in dem Untergestell eine Heizung nachgeordnet und außerdem auch die elektronische Steuereinheit in dem Untergestell angeordnet ist. Das Massagegerät braucht an seinem Bestimmungsort danach lediglich mit Strom versorgt, beispielsweise mittels eines Steckers an das Netz angeschlossen zu werden, um voll einsatzbereit zu sein.

Die Erfindung wird nachfolgend anhand des in der Zeichnung dargestellten Ausführungsbeispiels näher erläutert. Es zeigen:
- Fig. 1: ein aus einem Untergestell und einer Haube bestehendes Massagegerät in der Längsansicht;
- Fig. 2: das Massagegerät gemäß Fig. 1 von links gesehen;
- Fig. 3: eine erste Ausführung einer Haube in der Draufsicht und von innen gesehen;
- Fig. 4: eine zweite Ausführung einer Haube in der Draufsicht und von innen gesehen;
- Fig. 5: ein erfindungsgemäßes Massagegerät perspektivisch dargestellt;
- Fig. 6: als Einzelteil ein in das Untergestell eines erfindungsgemäßen Massagegerätes integrierbares Bauteil; und
- Fig. 7: die Haube gemäß Fig. 3 entlang der Linie VII-VII geschnitten.

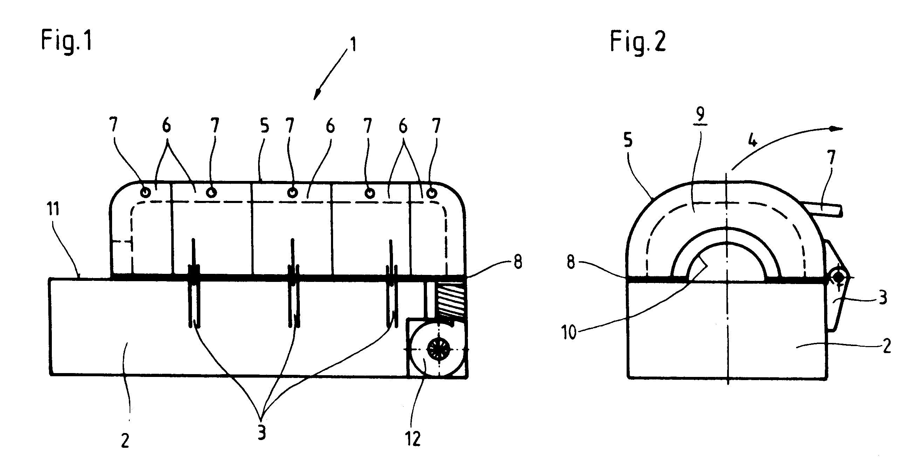

Ein Massagegerät 1 besteht aus einem mit einer Liegefläche versehenen Untergestell 2 und einer daran mittels Scharnieren 3 befestigten, in Pfeilrichtung 4 (vgl. Fig. 2) verschwenkbaren, halbschalenartigen Haube 5. Diese setzt sich aus mehreren Halbschalensegmenten 6 zusammen, von denen jedes einen separaten Luftanschluß 7 aufweist. In der in Fig. 1 dargestellten, geschlossenen Lage des Massagegerätes 1 dichtet eine umlaufende Dichtung 8 die Haube 5 gegen das beispielsweise mit einer Matratze oder einem Wasserbett als Liegefläche versehene Untergestell 2 ab.

Die Haube 5 ist kürzer als das Untergestell 2 und besitzt in ihrer vorderen Stirnwand 9 einen halbkreisförmigen Ausschnitt 10. Nach dem Schließen der Haube 5 schaut lediglich der Kopf einer zu behandelnden Person aus der Haube 5 des Massagegerätes 1 hervor und findet eine Auflage auf dem freien, gegenüber der Haube 5 vorkragenden Kopfteil 11 des Untergestells 2. In dem Untergestell 2 sind außerdem ein Gebläse 12 und eine diesem nachgeschaltete Heizung 13 angeordnet; die von dem Gebläse 12 angesaugte, von einem nicht dargestellten Filter vorgereinigte Umgebungsluft wird durch die nachgeschaltete Heizung erwärmt, bevor sie in das Innere der Haube 5 gelangt. Anstatt von dem Gebläse 12, können die Segmente 6 der Haube 5 über ihre Luftanschlüsse 7 von einer nicht dargestellten Druckmittelquelle, beispielsweise einem Kompressor mit Luft versorgt werden.

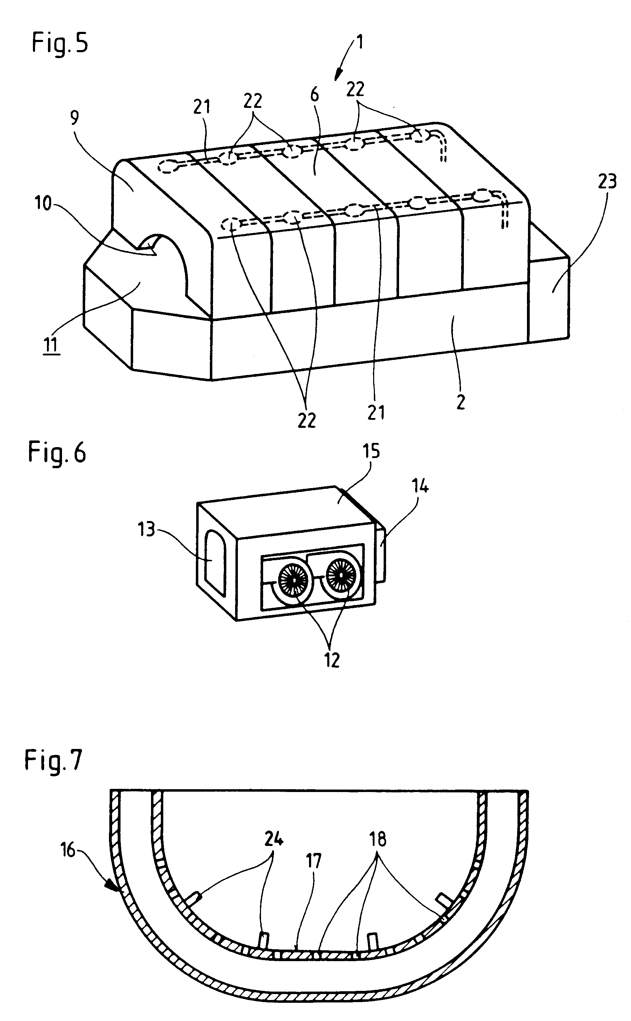

Wie in Fig. 6 dargestellt ist, können das bzw. die Gebläse 12 zusammen mit der Heizung 13 und einem Wirkstoffe wie Ozon, Kräuteressenzen oder Öle enthaltenden Behälter 14 in einem separaten Bauteil 15 angeordnet sein, das sich im Untergestell 2 integrieren bzw. dort unterbringen läßt. Sofern zusammen mit der Luft den Behandlungszweck unterstützende Wirkstoffe in das Innere der Haube 5 eingedüst werden sollen, empfiehlt es sich, den Wirkstoff-Behälter 14 so anzuordnen, daß die Wirkstoffe von dem Luftstrom erst mitgerissen werden, nachdem dieser beheizt wurde.

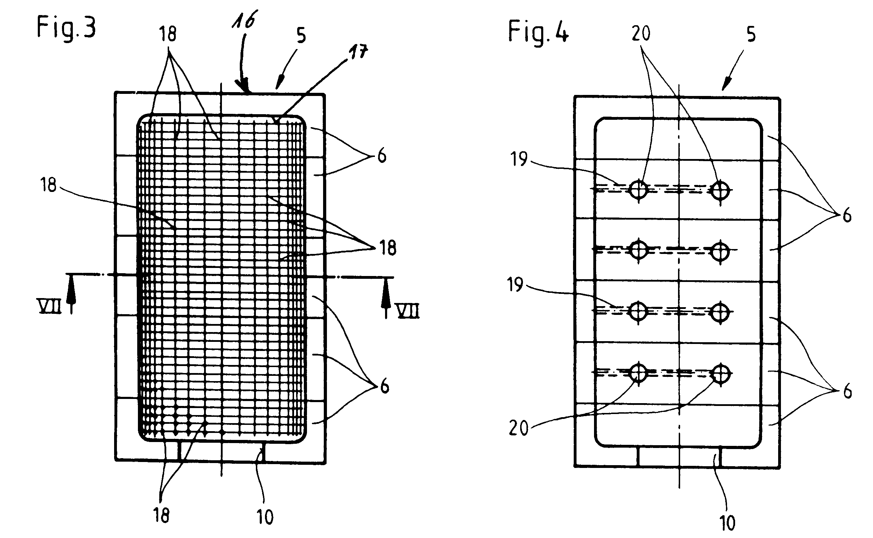

Die Haube 5 besitzt Hohlraumwände 16 (vgl. Fig. 7), von denen gemäß einer in Fig. 3 dargestellten ersten Ausführung die Innenwand 17 mit einer Vielzahl als Luftdüsen 18 ausgebildeten Bohrungen versehen ist. Damit ergibt sich eine große Verteilung der von dem Gebläse 12 angesaugten Luft und damit eine Unterstützung der aufgrund der Segmentbauweise der Haube 5 erreichten punktuellen Verteilung der Luftbedüsung. Bei der Ausführung der Haube 5 gemäß Fig. 4 sind die Halbschalensegmente 6 mit je zwei großdimensionierten, an einem Düsenbalken 19 befestigten Luftdüsen 20 versehen. Die Düsenbalken 19 verlaufen dort quer zur Längsrichtung der Haube 5, während sich gemäß Fig. 5 zwei Düsenbalken 21 mit Luftdüsen 22 in Längsrichtung der Haube 5 erstrecken.

Mittels einer im Untergestell 2 integrierten - gemäß Fig. 5 an dem dem Kopfteil abgewandten Ende des Untergestells 2 befestigten - elektronischen Steuereinheit 23 lassen sich die Luftdüsen 18 bzw. 20 bzw. 22 entweder selektiv, entsprechend ihren Segmenten 6 zonenweise oder alle gemeinsam ansteuern und sowohl die punktuelle Verteilung der Luftbedüsung als auch die Intensität der Luftströme beeinflussen. Außerdem kann mit der Steuereinheit 23 die Temperatur der über die Düsen 18, 20, 22 in das Innere der Haube 5 bzw. des Massagegerätes 1 eingeblasenen Luft gezielt verändert und den individuellen Wünschen und Therapiezwecken entsprechend angepaßt werden. Wie in Fig. 7 dargestellt ist, lassen sich die Düsen 18, 20, 22 mit in der Haube 5 angeordneten Lichtstrahlern 24 kombinieren.

## Patentansprüche

1. Massagegerät mit einer Haube (5) aus einer Hohlraumwandung (16), wovon die Innenwand (17) mit Luftdüsen (18, 20, 22) versehen ist, dadurch gekennzeichnet, daß die Luftdüsen (18, 20, 22) mit erwärmter Luft versorgt werden und die erwärmte Luft unmittlebar in den von der Hohlraumwandung (16) eingeschlossenen Hohlraum der Haube (5) geleitet wird.

2. Massagegerät nach Anspruch 1, dadurch gekennzeichnet, daß die Haube (5) mit stirnseitigen Wänden versehen ist und zumindest eine Stirnwand (9) einen Ausschnitt (10) aufweist.

3. Massagegerät nach Anspruch 1 oder 2, dadurch gekennzeichnet, daß die Haube (5) aus mehreren Halbschalen-Segmenten (6) besteht.

4. Massagegerät nach Anspruch 3, gekennzeichnet durch separate Luftanschlüsse (7) für jedes Segment (6).

5. Massagegerät nach einem oder mehreren der Ansprüche 1 bis 4, dadurch gekennzeichnet, daß in der Haube (5) Lichtstrahler (24) angeordnet sind.

6. Massagegerät nach einem oder mehreren der Ansprüche 1 bis 5, dadurch gekennzeichnet, daß die Luftdüsen (18, 20, 22) selektiv ansteuerbar sind.

7. Massagegerät nach einem oder mehreren der Ansprüche 1 bis 6, dadurch gekennzeichnet, daß die Temperatur der den Düsen (18, 20, 22) zugeführten Luft regelbar ist.

8. Massagegerät nach einem oder mehreren der Ansprüche 1 bis 7, dadurch gekennzeichnet, daß die Haube (5) schwenkbeweglich an einem Untergestell (2) befestigt ist.

9. Massagegerät nach Anspruch 8, dadurch gekennzeichnet, daß das Untergestell (2) als Liegefläche ein Wasserbett aufweist.

10. Massagegerät nach Anspruch 8 oder 9, dadurch gekennzeichnet, daß in dem Untergestell (2) zumindest ein Gebläse (12) angeordnet ist.

11. Massagegerät nach einem oder mehreren der Ansprüche 8 bis 10, gekennzeichnet durch eine dem Gebläse (12) in Richtung des Luftstroms nachgeordnete Heizung (13).

12. Massagegerät nach einem oder mehreren der Ansprüche 8 bis 11, gekennzeichnet durch eine im/am Untergestell (2) angeordnete elektronische Steuereinheit (23).

## Claims

1. A massage device comprising a hood (5) with a cavity wall (16) of which the inner wall (17) is provided with air nozzles (18, 20, 22), characterised in that the air nozzles (18, 20, 22) are supplied with heated air and the heated air is led directly into the cavity of the hood (5) enclosed within the cavity wall (16).

2. A massage device according to claim 1, characterised in that the hood (5) is provided with end walls and at least one end wall (9) has a cutout (10).

3. A massage device according to claim 1 or claim 2, characterised in that the hood (5) is made up of a plurality of half shell segments (6).

4. A massage device according to claim 3, characterised by having separate air connections (7) for each segment (6).

5. A massage device according to one or more of claims 1 to 4, characterised in that luminous radiators (24) are arranged in the hood (5).

6. A massage device according to one or more of claims 1 to 5, characterised in that the air nozzles (18, 20, 22) are selectively controllable.

7. A massage device according to one or more of claims 1 to 6, characterised in that the temperature of the air supplied to the nozzles (18, 20, 22) can be regulated.

8. A massage device according to one or more of claims 1 to 7, characterised in that the hood (5) is attached pivotably to a base (2).

9. A massage device according to claim 8, characterised in that the base (2) has a water bed as reclining surface.

10. A massage device according to claim 8 or claim 9, characterised in that at least one blower (12) is arranged in the base (2).

11. A massage device according to one or more of claims 8 to 10, characterised by having a heater (13) following the blower (12) in the direction of the air flow.

12. A massage device according to one or more of claims 8 to 11, characterised by having an electronic control unit (23) arranged in or on the base (2).

## Revendications

1. Appareil de massage avec un capot (5) formée d'une paroi creuse (16), dont la paroi intérieure (17) est pourvue de buses à air (12, 20, 22), caractérisé en ce que les buses à air (18, 20, 22) sont alimentées en air réchauffé et en ce que l'air réchauffé est admis directement dans la cavité du capot (5) enfermée par la paroi creuse (16).

2. Appareil de massage selon la revendication 1, caractérisé en ce que le capot (5) est pourvu de parois d'extrémité, et en ce que l'une au moins des parois d'extrémité (9) présente une découpe (10).

3. Appareil de massage selon l'une ou l'autre des revendications 1 et 2, caractérisé en ce que le capot (8) est constitué de plusieurs segments (6) en forme de demi-coquille.

4. Appareil de massage selon la revendication 3, caractérisé en ce que des raccords à air (7) séparés sont prévus pour chaque segment (6).

5. Appareil de massage selon l'une ou plusieurs des revendications 1 à 4, caractérisé en ce que des projecteurs lumineux (24) sont agencés dans le capot (5).

6. Appareil de massage selon l'une ou plusieurs des revendications 1 à 5, caractérisé en ce que les buses à air (18, 20, 22) peuvent être commandées de manière sélective.

7. Appareil de massage selon l'une ou plusieurs des revendications 1 à 6, caractérisé en ce que la température de l'air admis aux buses (18, 20, 22) est réglable.

8. Appareil de massage selon l'une ou plusieurs des revendications 1 à 7, caractérisé en ce que le capot (5) est fixé sur un châssis inférieur (2) de manière à pouvoir se déplacer en basculement.

9. Appareil de massage selon l'une ou plusieurs des revendications 1 à 8, caractérisé en ce que le châssis inférieur (2) comprend un matelas à eau en tant que surface de repos.

10. Appareil de massage selon l'une ou l'autre des revendications 8 ou 9, caractérisé en ce qu'au moins un ventilateur (12) est agencé dans le châssis inférieur (2).

11. Appareil de massage selon l'une ou plusieurs des revendications 8 à 10, caractérisé en ce qu'il comprend un dispositif de chauffage (13) agencé en aval du ventilateur (12) dans la direction d'écoulement d'air.

12. Appareil de massage selon l'une ou plusieurs des revendications 8 à 11, caractérisé en ce qu'il comprend une unité de commande électronique (23) agencée dans et/ou sur le châssis inférieur (2).
